(19) Europäisches Patentamt
European Patent Office
Office européen des brevets

(11) Publication number: **0 226 272 B1**

(12) # EUROPEAN PATENT SPECIFICATION

(45) Date of publication of patent specification: **06.03.91** (51) Int. Cl.$^5$: **G01N 1/30**

(21) Application number: **86306853.2**

(22) Date of filing: **04.09.86**

(54) **Detection of reticulocytes.**

(30) Priority: **01.11.85 US 793813**

(43) Date of publication of application:
**24.06.87 Bulletin 87/26**

(45) Publication of the grant of the patent:
**06.03.91 Bulletin 91/10**

(84) Designated Contracting States:
**BE CH DE FR GB IT LI NL SE**

(56) References cited:
**EP-A- 0 114 462**
**US-A- 3 872 312**
**US-A- 4 331 759**
**US-A- 4 424 201**

(73) Proprietor: **BECTON, DICKINSON & COMPANY**
**One Becton Drive**
**Franklin Lakes New Jersey 07417-1880(US)**

(72) Inventor: **Lee, Linda G.**
**225, Waverley Street No. 4**
**Menlo Park California(US)**
Inventor: **Chen, Chia-Huei**
**1182 Royston Court**
**San Jose California(US)**

(74) Representative: **Ruffles, Graham Keith et al**
**MARKS & CLERK 57-60 Lincoln's Inn Fields**
**London WC2A 3LS(GB)**

EP 0 226 272 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention generally relates to the detection and enumeration of reticulocytes in a blood sample. More particularly, the present invention relates to a dye which is suitable for staining ribonucleic acid and ribonucleic acid polymers (RNA) and is particularly suitable for staining reticulocytes. The invention further relates to a fluorescent composition.

In many cases, there is a need to detect RNA or RNA-containing substances. Thus, for example, reticulocytes are known to contain RNA, and detection and enumeration of reticulocytes in a blood sample is of value to clinicians. The reticulocyte count of a blood sample is used as an indicator of erythropoietic activity, has diagnostic and prognostic value in acute hemorrhage and hemolytic anemia, and is a measure of response to iron, vitamin $B_{12}$ and folic acid therapy. As known in the art, reticulocytes are precursors to mature red blood cells, and hence the term reticulocyte embraces the evolution and development of the cell whereby a mature red blood cell is generated.

In the past, reticulocytes in a blood sample have been determined by both manual and automated methods by using appropriate stains such as new methylene blue (NMB), brilliant cresyl blue (BCB), acridine orange and pyronin Y.

Vital staining with the dye new methylene blue is considered to be the reference method for reticulocyte determinations, and in use this dye precipitates RNA. The method is manual, requires counting large numbers (for example, 500 to 1,000) of cells with a microscope, is slow, tedious, and subject to errors. New methylene blue is nonfluorescent and true precipitated RNA is often difficult to differentiate from precipitated stain.

Acridine orange has had some use in staining reticulocytes by both manual and automated procedures. Acridine orange also precipitates RNA, and this prevents quantitative estimates of RNA content because of potential quenching. Moreover, acridine orange does not lead to a diffuse fluorescent distribution of stained cells. Age profiles of the cells (based on RNA content being proportional to fluorescence) are not reliable. Acridine orange has a great affinity for the plastic tubing in flow cytometers which leads to increased background and lengthy procedures for removing the dye from the flow cytometer tubing. In addition, acridine orange stained cells are difficult to separate from the autofluorescent red cell peak, and the reticulocyte count is usually lower than that obtained with new methylene blue.

The use of pyronin Y requires prior fixation of the erythrocytes with formalin, is cumbersome, time consuming, and generally yields poor results. Moreover, pyronin Y has a very low quantum efficiency, leading to very low fluorescent signals.

United States Patent Application Serial No. 460,144 filed January 24, 1983, relates to a method for detecting reticulocytes utilizing thioflavin T as a dye for staining reticulocytes.

A dye for staining reticulocytes preferably has the following properties:

1. The dye should not fluoresce in the absence of RNA.
2. The dye should have a good fluorescence quantum yield.
3. The dye must be able to penetrate the membrane of cells containing RNA.
4. The dye should preferably have an excitation peak at about 488 nm.

None of the aforementioned known dyes for RNA and reticulocytes have all of the desirable features described hereinabove.

Accordingly, there is need for providing a dye better suited for staining reticulocytes so as to provide a procedure for accurately determining reticulocytes in a blood sample.

In accordance with one aspect of the present invention, there is provided an improvement in a process for detecting reticulocytes wherein the reticulocytes are stained with a thiazole orange dye. Such a dye has the following formula:

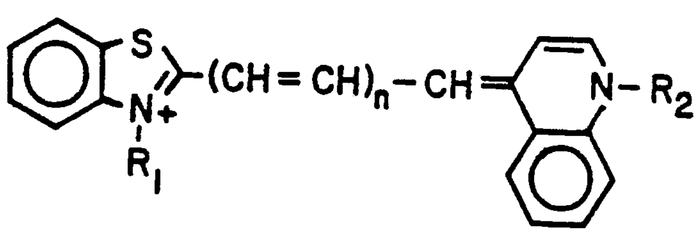

wherein: $R^1$ represents an alkyl group having from 1 to 6 carbons;
$R^2$ represents an alkyl group having from 1 to 6 carbons; and
n is zero or an integer from 1 to 6.

In accordance with another aspect of the present invention, reticulocytes are detected in a flow cytometer after the reticulocytes have been stained with the dye of the invention.

In accordance with a further aspect of the present invention, there is provided a composition comprised of reticulocytes stained with the dye of the invention.

The dye of invention differs structurally from thioflavin T. Thioflavin T has the following structure:

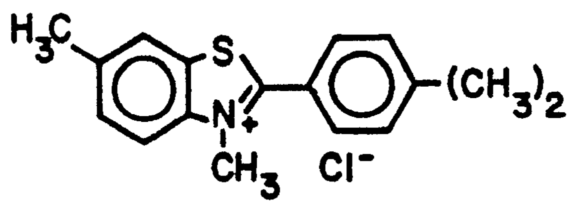

For convenience, the dye of the invention for staining reticulocytes is referred to as "athiazole orange".

Applicant has found that thiazole orange is an effective dye for staining reticulocytes. The use of thiazole orange offers the further advantage that thiazole orange when unbound to ribonucleic acid provides little or no fluorescence, whereas thiazole orange when bound to ribonucleic acid in the reticulocytes is fluorescent. Thiazole orange can be excited at 488 nm whereas thioflavin T is excited at a maximum of about 455 nm.

In accordance with the present invention, when staining reticulocytes in a blood sample, thiazole orange may be employed as an aqueous solution, and in particular as an isotonic saline solution, which may contain a minor amount of methanol. The blood sample, which may be whole blood or a blood fraction, is stained with the thiazole orange by mixing the blood sample with the solution of thiazole orange. It has been found that by using thiazole orange as the stain, it is possible to detect and enumerate reticulocytes in a whole blood sample.

Thiazole orange exhibits a strong absorption peak (unbound) at about 470 nm; however in the unbound state, the dye does not provide either a detectable excitation or emission peak. When thiazole orange stains the RNA in the reticulocytes, the optical properties thereof change dramatically. In particular, the absorption curve shifts to a longer wavelength, and the dye now exhibits strong fluorescence. The excitation maximum is at about 510 nm, and the emission maximum is at about 530 nm, giving a Stokes shift of about 20 nm. As a result of the excitation peak of the bound thiazole orange being in the order of about 510 nm, in using the automatic flow cytometer the light source may be a mercury lamp which has an energy line at about 485 nm or an argon ion laser which has strong emission at 488 nm. The lack of fluorescence of the thiazole orange dye when not bound to nucleic acid provides low background and allows an operator to select a fluorescent threshold (or "gates") for an automatic flow cytometer by simply running an unstained control. Although excitation may be effected at other wavelengths, reticulocytes stained with thiazole orange are preferably excited at a wavelength of from about 460 nm to about 520 nm.

Thiazole orange dye does not precipitate RNA, and as a result, reticulocytes stained with thiazole orange maintain a relatively homogeneous distribution of intracellular RNA, whereby there is a nearly linear relationship between the fluorescent signal measured for an individual reticulocyte and its RNA content. Clinically, this provides the physician with additional information beyond the reticulocyte count in that RNA content is a function of reticulocyte age. Accordingly, by using thiazole orange, a clinician has the ability to do reticulocyte age profiles as well as simple reticulocyte counts.

The use of thiazole orange for staining reticulocytes in a blood sample offers the further advantage that the fluorescent signals from the stained reticulocytes are well separated from those of the mature erythrocytes, whereby results can be directly read in an automatic flow cytometer without extensive data manipulation.

Reticulocytes stained with thiazole orange, although preferably enumerated in an automatic flow cytometer, can also be counted by a manual procedure or automated microscopy.

Automatic flow cytometers are well known in the art, and the present invention is not limited to the use of any particular flow cytometer. Thus, for example, reticulocytes stained with thiazole orange may be detected and enumerated in the FACS 440™ flow cytometer for the FACS Analyzer™ flow cytometer, both sold by Becton Dickinson and Company. In using such automatic flow cytometers, fluorescent gates are set

by use of an unstained control sample, and the fluorescent gates are then used on the stained sample.

The use of an automatic flow cytometer for detection and enumeration of reticulocytes stained with thiazole orange provides results which closely correlate with results obtained by a known standard method for enumerating reticulocytes which uses methylene blue or acridine orange.

The use of reticulocytes stained with thiazole orange in an automatic flow cytometer is particularly advantageous in that there are low fluorescence background and flourescent gates may be easily selected by use of an unstained control. Moreover, there is no precipitation of intracellular reticulocyte RNA, whereby the cells need not be fixed. In addition, there is a linear relationship between the fluorescent signal for an individual reticulocyte, which provides information as to reticulocyte age.

Still another advantage of the present invention is that reticulocytes stained with thiazole orange can be used in a automatic flow cytometer having lower sensitivities, e.g., one may use a mercury arc lamp as opposed to an argon laser.

Flow Cytometry and Sorting , pages 457-58 Edited by Melamed et al., John Wiley & Sons, describes the use of both acridine orange and thioflavin T for staining RNA of living cells, this publication does not disclose the use of thiazole orange as a stain for reticulocyte detection and enumeration in an automatic flow cytometer.

The following non-limiting example illustrates various features of the present invention.

## EXAMPLE

Thiazole orange wherein $R^1$ and $R^2$ represent a methyl group and n is 1 was used in a procedure for reticulocyte staining.

A 1 mg/ml stock solution of the dye in methanol was prepared. A 1:8,000 dilution of the stock solution in pH 7.2 phosphate buffered saline was made, 5 $\mu$l of whole blood was mixed into 1 ml of the diluted dye solution. The sample was analyzed on a FACS 440TM flow cytometer with an excitation wavelength of 488 nm and 530/30 nm bandpass filter.

Figures 1 to 5 show FACS data for reticulocyte analysis of normal and anemic blood using thiazole orange of this example. Figure 1 shows a fluorescene histogram and fluorescence vs. forward scatter for normal, unstained blood. Figure 2 shows data for normal blood stained with thiazole orange. Figure 3 shows unstained anemic blood (17.3% reticulocytes by new methylene blue assay); Figure 4 shows the anemic blood after staining. Figure 5 shows samples of anemic blood (8.4% reticulocytes by new methylene blue assay) stained with thiazole orange of this example for varying lengths of time (30 minutes, 70 minutes, 7 hours).

Numerous modifications and variations of the present invention are possible in light of the above teachings, and, therefore the invention may be practiced otherwise than as particularly described.

## Claims

1. A process for detecting reticulocytes by staining with a dye characterized in that the reticulocytes are detected by staining with a thiazole orange.

2. A process according to Claim 1, wherein the detecting is effected in a flow cytometer.

3. A process according to Claim 2, wherein the reticulocytes stained with thiazole orange are excited in the flow cytometer with light from a mercury arc lamp.

4. A process according to Claim 2, wherein the reticulocytes stained with thiazole orange are excited in the flow cytometer with light from an argon laser.

5. A process according to any preceding Claim, wherein the reticulocytes are present in a sample comprising whole blood.

6. A process according to any preceding Claim, wherein the reticulocytes are detected without fixation thereof.

7. A composition comprising reticulocytes stained with a thiazole orange.

**Revendications**

1. Procédé de détection de réticulocytes par coloration au moyen d'un colorant, caractérisé en ce qu'on détecte les réticulocytes en les colorant au moyen d'un orangé de thi-azole.

2. Procédé selon la revendication 1, caractérisé en ce qu'on réalise la détection dans un débit-cytomètre.

3. Procédé selon la revendication 2, caractérisé en ce qu'on excite les réticulocytes colorés à l'orangé de thia-zole, dans le débit-cytomètre, au moyen d'une lumière provenant d'une lampe à arc à mercure.

4. Procédé selon la revendication 2, caractérisé en ce qu'on excite les réticulocytes colorés à l'orangé de thia-zole, dans le débit-cytomètre, au moyen d'une lumière provenant d'un laser à l'argon.

5. Procédé selon l'une quelconque des revendications pré-cédentes, caractérisé en ce que les réticulocytes sont contenus dans un échantillon contenant du sang entier.

6. Procédé selon l'une quelconque des revendications pré-cédentes, caractérisé en ce qu'on détecte les réticulocy-tes sans les fixer.

7. Composition comprenant des réticulocytes colorés à l'orangé de thiazole.

**Ansprüche**

1. Verfahren zum Nachweisen von Retikulozyten durch Färben mittels eines Farbstoffes, dadurch gekennzeichnet, daß die Retikulozyten durch Färben mit einem Thiazol Orange nachgewiesen werden.

2. Verfahren nach Anspruch 1, wobei der Nachweis in einem Strömunaszytometer vorgenommen wird.

3. Verfahren nach Anspruch 2, wobei die mit Thiazol Orange Gefärbten Retikulozyten in einem Strömunaszytometer mit Licht aus einer Quecksilberbogenlampe angeregt werden.

4. Verfahren nach Anspruch 2, wobei die mit Thiazol Orange gefärbten Retikulozyten in einem Strömungszytometer mit Licht aus einer Argon-Laser angeregt werden.

5. Verfahren nach einem der vorhergehenden Ansprüche, wobei die Retikulozyten in einer Vollblut enthaltenden Probe vorhanden sind.

6. Verfahren nach einem der vorhergehenden Ansprüche, wobei die Retikulozyten ohne deren Fixierung nachgewiesen werden.

7. Zusammensetzung Retikulozyten umfassend, die mit einem Thiazol Orange gefärbt sind.

# FIG.1. Normal, unstained blood

BDIS CONSORT 30 - Rev D 09/85

Sample : SAMPLE 001 Date: 21-May-85 16:46
Cytometer: FACS 440 File: 052185001

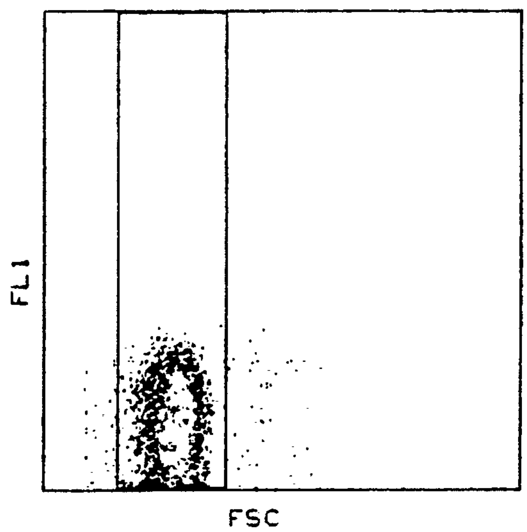


**Manual count = 1.9 %**

FSC 39, 57
FL1 0, 255
FL2 0, 255
SSC 0, 255

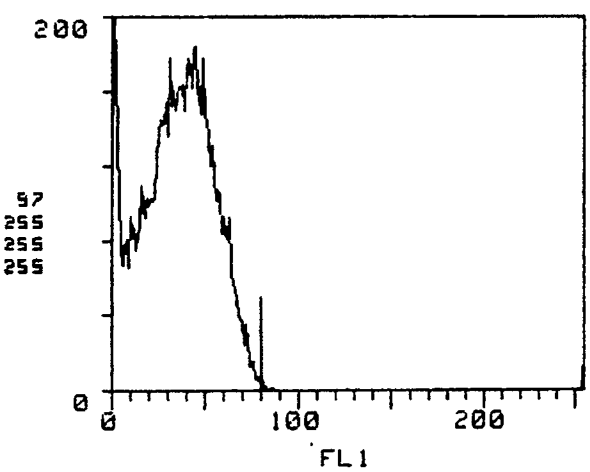


Single histogram statistics

Parameter : FL1 Gated events : 9863 Total events : 10000

| Left | Right | Events | % Gated | % Tot | Mean | Mode | Peak | CV |
|---|---|---|---|---|---|---|---|---|
| 0 | 79 | 9823 | 99.6 | 98.2 | 30.29 | 0.00 | 1293 | 69.6 |
| 80 | 255 | 40 | 0.4 | 0.4 | 190.10 | 255.00 | 25 | 44.6 |

**FIG.2. Normal blood stained with thiazole orange**

BDIS CONSORT 30 - Rev D 09/85

Sample : SAMPLE 004
Cytometer: FACS 440

Date: 21-May-85 16:48
File: 052185004

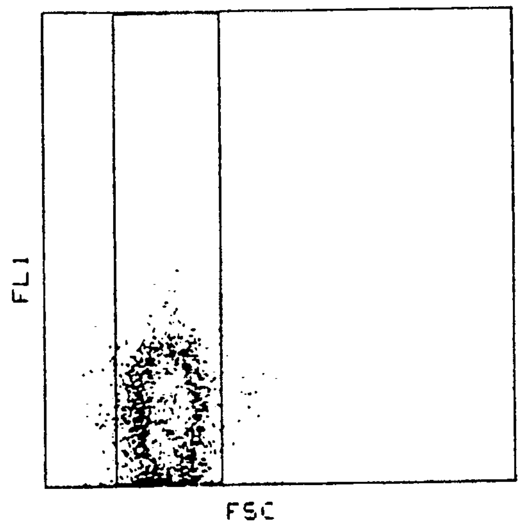


**FACS 440™ count = 1.2 %**
**Manual count = 1.9 %**

FSC 39. 97
FL1 0.255
FL2 0.255
SSC 0.255

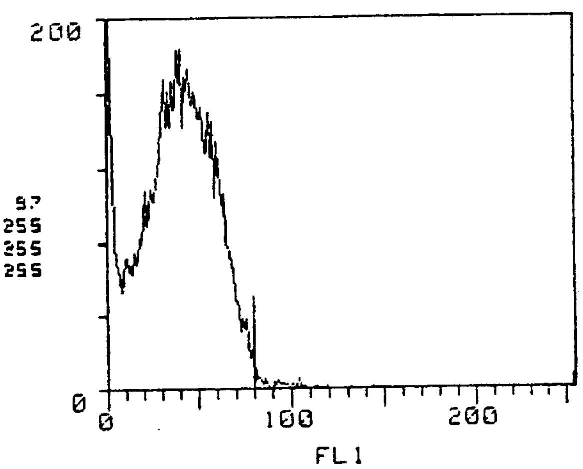


Single histogram statistics

Parameter : FL1    Gated events : 9781    Total events : 10000

| Left | Right | Events | % Gated | % Tot | Mean | Mode | Peak | CV |
|---|---|---|---|---|---|---|---|---|
| 0 | 79 | 9659 | 98.8 | 96.6 | 33.75 | 0.00 | 1130 | 65.2 |
| 80 | 255 | 122 | 1.2 | 1.2 | 112.73 | 80.00 | 17 | 48.8 |

# FIG.3. Anemic blood, unstained

BDIS CONSORT 30 - Rev D 09/85

Sample : SAMPLE 002
Cytometer: FACS 440

Date: 21-May-85 16:47
File: 052185002

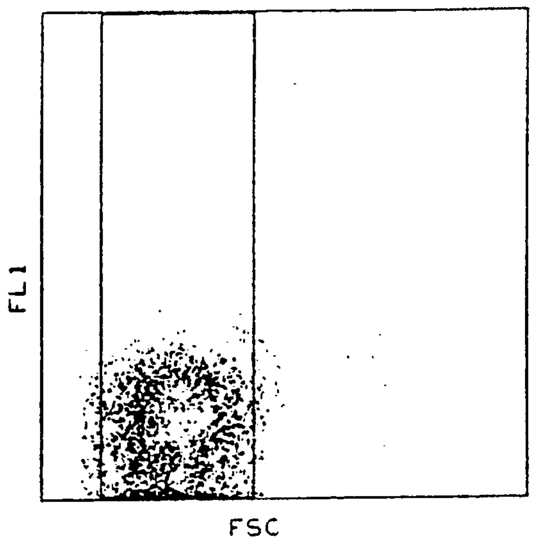


Manual count = 17.3 %

FSC 31,112
FL1 0,255
FL2 0,255
SSC 0,255

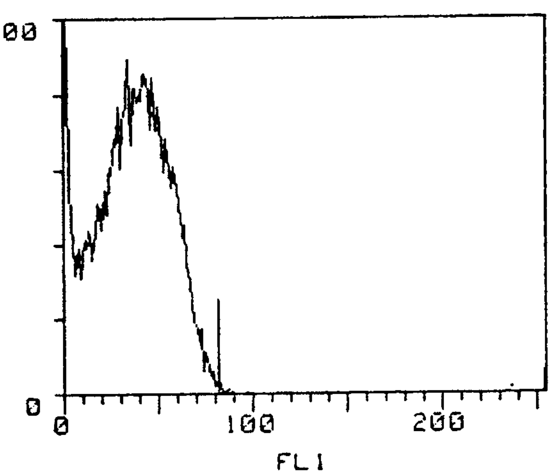


Single histogram statistics

Parameter : FL1 Gated events : 9684 Total events : 10000

| Left | Right | Events | % Gated | % Tot | Mean | Mode | Peak | CV |
|---|---|---|---|---|---|---|---|---|
| 0 | 81 | 9636 | 99.5 | 96.4 | 31.61 | 0.00 | 1230 | 68.2 |
| 82 | 255 | 48 | 0.5 | 0.5 | 173.96 | 255.00 | 25 | 49.1 |

FIG.4. Anemic blood stained with thiazole orange

BDIS CONSORT 30 - Rev D 09/85

Sample : SAMPLE 005
Cytometer: FACS 440

Date: 21-May-85 16:50
File: 052185005

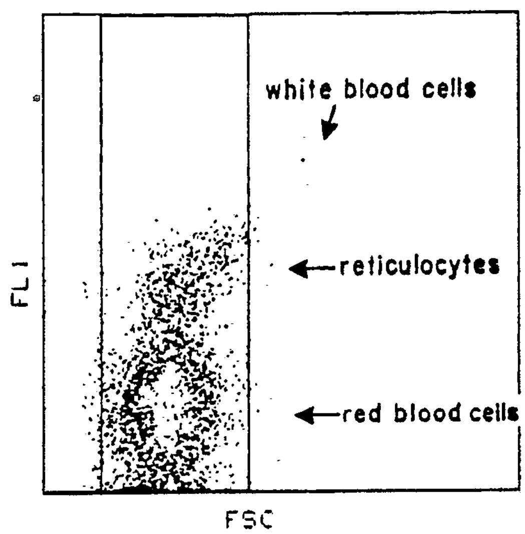


FACS 440™ count = 16.0 %
Manual count = 17.3 %

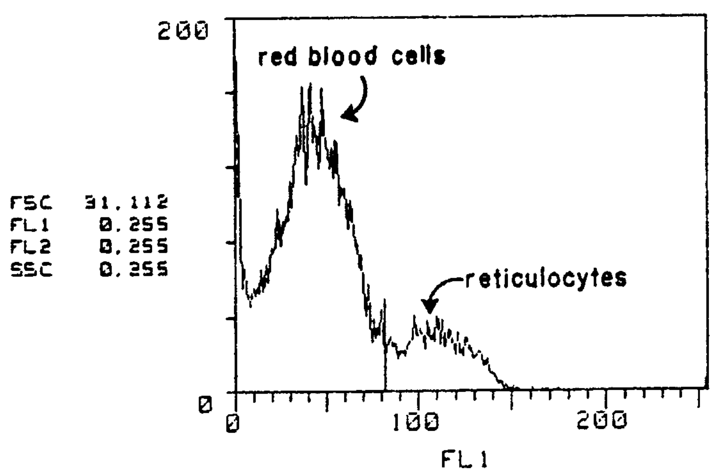


Single histogram statistics

Parameter : FL1 Gated events : 9710 Total events : 10000

| Left | Right | Events | % Gated | % Tot | Mean | Mode | Peak | CV |
|---|---|---|---|---|---|---|---|---|
| 0 | 81 | 8153 | 84.0 | 81.5 | 36.39 | 0.00 | 779 | 61.5 |
| 82 | 255 | 1557 | 16.0 | 15.6 | 111.60 | 98.00 | 41 | 19.1 |

FIG.5. Anemic blood stained with thiazole orange for 30 minutes, 70 minutes and 7 hours.

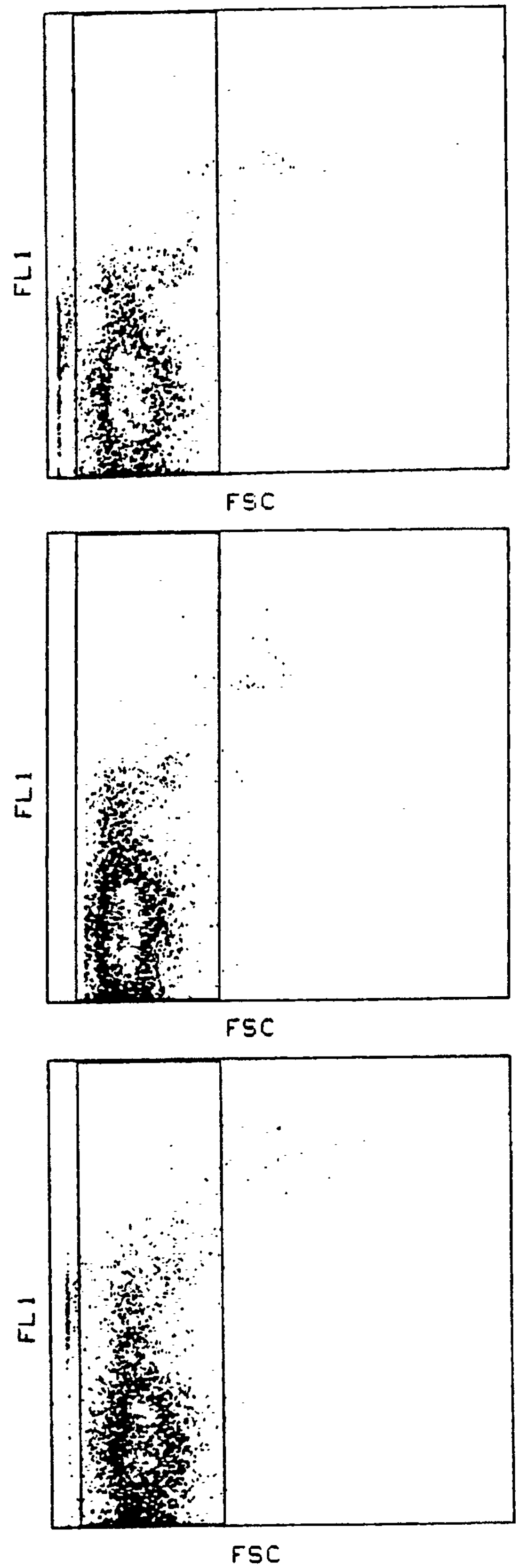


Manual count = 8.4 %

30 minutes,
6.8 % reticulocytes

70 minutes,
7.6 % reticulocytes

7 hours,
8.9 % reticulocytes